# EUROPEAN PATENT SPECIFICATION

(11) **EP 3 020 375 B1**
(45) Date of publication and mention of the grant of the patent: **09.01.2019**
(21) Application number: 15194277.8
(22) Date of filing: 12.11.2015
(51) Int. Cl.: A61F 2/44

(54) **SPINAL FIXATION DEVICE**
WIRBELSÄULENFIXIERUNGSVORRICHTUNG
DISPOSITIF DE FIXATION VERTÉBRALE

(30) Priority: 12.11.2014 US 201462078666 P
(43) Date of publication of application: 18.05.2016
(73) Proprietor: K2M, Inc., Leesburg, VA 20175 (US)
(72) Inventor: Sutterlin, III Chester E., Longboat Key, Florida 32610 (US); Mendel, Ehud, Columbus, Ohio 43209 (US); Finn, Michael, Aurora, Colorado 80045 (US); Boyd, Clint, Winchester, Virginia 22601 (US); Jones, Scott, McMurray, Pennsylvania 15317 (US); Wallenstein, Todd, Ashburn, Virginia 20147 (US); Buchowski, Jacob M., St. Louis, MO 63124 (US)
(74) Representative: Hoffmann Eitle

(56) References cited:
- EP-A1- 1 878 408
- US-A1- 2014 277 503

## Description

### BACKGROUND

### Technical Field

The present disclosure relates to an apparatus for treating spinal conditions, and more particularly, to an intervertebral implant.

### Background of Related Art

The human spine includes thirty-three vertebrae. The vertebrae interlock with one another to form a spinal column. Each vertebra has a cylindrical bony body (vertebral body), two pedicles extending from the vertebral body, a lamina extending from the pedicles, two wing-like projections extending from the pedicles, a spinous process extending from the lamina, a pars interarticularis, two superior facets extending from the pedicles, and two inferior facets extending from the lamina. The vertebrae are separated and cushioned by thin pads of tough, resilient fiber known as inter-vertebral discs. Inter-vertebral discs provide flexibility to the spine and act as shock absorbers during activity. A small opening (foramen) located in each vertebra allows passage of the spinal cord. When the vertebrae are properly aligned, the spinal cord passes through without a problem. However, when the vertebrae are misaligned or a constriction is formed in the spinal canal, nerves of the spinal cord may get compressed and may cause back pain, leg pain, or other neurological disorders.

Disorders of the spine that may cause misalignment of the vertebrae or constriction of the spinal canal include spinal injuries, infections, tumor formation, herniation of the inter-vertebral discs (i.e., slippage or protrusion), arthritic disorders, and scoliosis. In these pathologic circumstances, surgery may be tried to either decompress the neural elements and/or fuse adjacent vertebral segments. Decompression may involve laminectomy, discectomy, or corpectomy. Laminectomy involves the removal of part of the lamina, i.e., the bony roof of the spinal canal. Discectomy involves removal of the inter-vertebral discs. Corpectomy involves removal of the vertebral body as well as the adjacent inter-vertebral discs.

A number of spinal surgical devices may be used to promote bony fusion after decompressing the spinal nerves. For instance, surgeons often replace the diseased vertebral tissue with one or more spinal cages and bone support matrix. Spinal cages support adjacent vertebral segments, while furthering spinal fusion of adjacent vertebral bodies. Scientists and clinicians have developed a number of devices and methods for decompressing spinal nerves. Improvements to these methods and devices are nevertheless still possible. Reference may be made to U.S. Patent Publication No. 2014/0277503 filed on March 14, 2014, entitled "Spinal Fixation Device," for a detailed discussion of the construction and operation of a spinal fixation system and an instrumentation for use therewith.

Furthermore, intervertebral spacer implants used as a stand-alone device or provided in an assembly including a retention mechanism to help alleviate expulsion and movement of the implant when placed in the spine, are well known. Such implant assemblies are advantageous in providing an implant that is easier to insert in the spine. Intervertebral spacer implant assemblies which include a spacer and a plate, where the plate comprises a supplemental or alternative retention mechanism having one or more holes in the anterior end of the plate that are directed toward the superior, inferior or both end plates of adjacent vertebrae are also known in the art. Such implants are used to stabilize and immobilize the spinal segments in the treatment of single or multi-level degenerative disc disease, spinal stenosis, and failed previous fusions, as well as other spine conditions.

To meet the problem of preventing expulsion of the interbody device and for providing stability to the anatomy, a need exists for an spinal fixation device that can be secured to the spine and provide anterior column support and stabilization, while providing a maximum fusion area.

Documents cited during prosecution include US 2014/0277503 A1, which discloses a spinal fixation device, upon which document the two-part form of Claim 1 is based.

### SUMMARY

In accordance with an embodiment of the present disclosure, there is provided a spinal fixation device comprising: an outer housing defining an aperture and a longitudinal axis; and an end plate assembly coupled with the outer housing, at least a portion of the end plate assembly slidably received within the outer housing, the end plate assembly including a first end plate configured to engage a vertebral body, wherein the end plate assembly is selectively movable between a first position in which the first end plate is spaced apart from the outer housing and a second position in which the first end plate is adjacent the outer housing, and the first end plate is selectively adjustable to an angular orientation of a plurality of angular orientations with respect to the longitudinal axis of the outer housing, wherein the end plate assembly includes first and second elongate members operatively coupled to the first end plate such that axial movement of the first and second elongate members in tandem causes axial displacement of the first end plate, and relative movement between the first and second elongate members transitions the first end plate from a first angular orientation to a second angular orientation, and wherein the spinal fixation device further comprises a mounting assembly operatively supporting the first and second elongate members in the outer housing, characterized in that the mounting assembly includes a retaining housing and first and second rotatable members rotatably supported in the retaining housing, the first and second rotatable members operatively coupled with the first and second elongate members of the end plate assembly.

The mounting assembly may be releasably secured with the outer housing such that the end plate assembly is configured as a modular assembly interchangeably mounted in the outer housing. In particular configured so as to allow a clinician to interchange the end plate assembly with another end plate assembly.

The mounting assembly may be releasably secured with the outer housing. The first and second rotatable members of the mounting assembly may be spaced apart from each other to define a gap therebetween.

The device may comprise a spring pin received through a bore in the outer housing and through a transverse groove of the retaining housing to secure the retaining housing with the outer housing, such that when end plate assembly is pulled away from the outer housing, the spring pin flexes and disengages from the transverse groove to release the mounting assembly.

In an embodiment, each of the first and second rotatable members of the mounting assembly may include circumferentially arranged teeth opposing each other. The first rotatable member of the mounting assembly may include inner threads in a first orientation and the second rotatable member of the mounting assembly may include inner threads in a second orientation opposite to the first orientation.

The outer housing may define a plurality of bores. In particular, at least one bore of the plurality of bores may be in communication with the gap defined between the first and second rotatable members of the mounting assembly. The plurality of bores may be circumferentially arranged about the outer housing. The plurality of bores may be arranged along a length of the outer housing.

In accordance with another embodiment of present disclosure, there is provided a surgical kit including a spinal fixation device and a surgical instrument or a rod connector or a drug delivery assembly or a shield as defined below. The spinal fixation device includes an outer housing and an end plate assembly. The end plate assembly is coupled with the outer housing. At least a portion of the end plate assembly is slidably received within the outer housing through an aperture of the outer housing. The end plate assembly includes a first end plate configured to engage a vertebral body, wherein the end plate assembly is selectively movable between a first position in which the first end plate is spaced apart from the outer housing and a second position in which the first end plate is adjacent the outer housing. In addition, the first end plate is selectively adjustable to an angular orientation of a plurality of angular orientations with respect to the outer housing.

In an embodiment, the surgical instrument defines a channel extending therethrough. The surgical instrument defines an engaging portion configured to securely engage the outer housing of the spinal fixation device.

In an embodiment, the surgical instrument may include a height adjusting driver rotatably extending through the channel of the surgical instrument. The height adjusting driver may include an engaging portion having teeth configured to engage the circumferentially arranged teeth of the first and second rotatable members of the mounting assembly, whereby rotation of the height adjusting driver causes rotation of the first and second rotatable members of the mounting assembly.

In another embodiment, the surgical instrument may further include an angle adjusting driver rotatably extending through the channel of the surgical instrument. The angle adjusting driver may include an engaging portion having teeth configured to engage the circumferentially arranged teeth of one of the first or second rotatable members of the mounting assembly, whereby rotation of the angle adjusting driver causes rotation of the one of the first or second rotatable members of the mounting assembly.

In yet another embodiment, the kit may include or further include a rod connector having an anchoring portion coupled with the outer housing of the spinal fixation device and a head portion having a recessed portion configured to receive a spinal rod. In particular, the rod connector may define a channel therethrough. In addition, the head portion of the rod connector may define a bore in communication with the channel. The rod connector may include the anchoring portion, a coupling portion rotatably and pivotably coupled with anchoring portion and a head portion.

The anchoring portion may include an engaging portion threadably coupled with a bore of the outer housing.

The anchoring portion and coupling portion may be coupled by ball and socket joint.

The head portion may define slits configured to flex or enlarge the dimensions of recessed portion to facilitate insertion of the spinal rod.

The head portion may further define a bore and a screw so that upon insertion of the spinal rod in the recessed portion, the screw is able to be fastened to secure the spinal rod in the recessed portion.

In yet another embodiment, the kit may further include a drug delivery assembly including a drug reservoir securely coupled with the outer housing of the spinal fixation device, a supply port, and a catheter in fluid communication with the supply port and the drug reservoir.

In yet another embodiment, the kit may further include a shield attachable to the spinal fixation device. In particular, the shield may be configured to reduce radiation to a spinal cord. Alternatively, the shield may include slow releasing medicament.

### BRIEF DESCRIPTION OF THE DRAWINGS

In order to better understand the present invention, and to show how the same may be carried into effect, reference will now be made, by way of example only, to the accompanying drawings, in which:
FIG. 1 is a perspective view of a spinal fixation device in accordance with an embodiment of the present disclosure;
FIG. 2 is a rear view of the spinal fixation device of FIG. 1;
FIG. 3 is a side view of the spinal fixation device of FIG. 1;
FIG. 4 is a front view of the spinal fixation device of FIG. 1;
FIG. 5A is a bottom view of the spinal fixation device of FIG. 1;
FIG. 5B is a top view of the spinal fixation device of FIG. 1;
FIG. 6 is a exploded, perspective view of the spinal fixation device of FIG. 1 with parts separated;
FIG. 7 is a perspective view of the spinal fixation device of FIG. 1 with a first end plate spaced apart from a body of the spinal fixation device;
FIG. 8 is a rear view of the spinal fixation device of FIG. 7;
FIG. 9 is a cross-sectional view of the spinal fixation device of FIG. 8 cut along section line "9-9" of FIG. 8;
FIG. 10 is a perspective view of an insertion instrument illustrating use with the spinal fixation device of FIG. 1;
FIG. 11 is a top view of the insertion instrument of FIG. 10;
FIG. 12 is a perspective view of the insertion instrument of FIG. 10;
FIG. 13A is a side view of a height adjusting driver for use with the insertion instrument of FIG. 10;
FIG. 13B is a top view of the height adjusting driver of FIG. 13A;
FIG. 13C is a partial side view of a distal portion of the height adjusting driver of FIG. 13A;
FIG. 14A is a side view of an angle adjusting driver for use with the insertion instrument of FIG. 10;
FIG. 14B is a top view of the angle adjusting driver of FIG. 14A;
FIG. 14C is a partial side view of a distal portion of the angle adjusting driver of FIG. 14A;
FIG. 15A is a side view of a tapered driver for use with the insertion instrument of FIG. 10;
FIG. 15B is a top view of the tapered driver of FIG. 15A;
FIG. 15C is a partial side view of a distal portion of the tapered driver of FIG. 15A;
FIG. 16 is a top view of the insertion instrument of FIG. 10 illustrating use with the height adjusting driver of FIG. 13A with the spinal fixation device of FIG. 1;
FIG. 17 is a cross-sectional view of the insertion instrument and the spinal fixation device of FIG. 16 cut along section line "17-17" of FIG. 16;
FIG. 18 is a top view of the insertion instrument of FIG. 10 illustrating use with the angle adjusting driver of FIG. 14A and the spinal fixation device of FIG. 1; and
FIG. 19 is a cross-sectional view of the insertion instrument of FIG. 18 cut along section line "19-19" of FIG. 18;
FIG. 20a is a perspective view of a rod connector for use with the spinal fixation device of FIG. 1;
FIG. 20b is a perspective view of the rod connector of FIG. 20a illustrating polyaxial rotation thereof;
FIG. 21a is a perspective view of the spinal fixation device of FIG. 1 and the rod connector of FIG. 20a;
FIG. 21b is a perspective view of the spinal fixation device and the rod connector of FIG. 21a illustrating polyaxial rotation of the rod connector;
FIG. 22 is a perspective view of the spinal fixation device and the rod connector of FIG. 21a secured with the spinal fixation device illustrating use with a spinal rod;
FIG. 23 is a perspective view of the spinal fixation device of FIG. 22 secured between vertebral bodies by the rod connector and the spinal rod of FIG. 22;
FIG. 24 is a perspective view of the spinal fixation device of FIG. 22 secured between the vertebral bodies by a pair of rod connectors and spinal rods of FIG. 22;
IG. 25 is a perspective view of the spinal fixation device of FIG. 1 and a drug delivery assembly operatively coupled with the spinal fixation device;
FIG. 26 is a perspective view of the spinal fixation device of FIG. 23 and a drug delivery assembly in accordance with another embodiment of the present disclosure;
FIG. 27 is a perspective view of the spinal fixation device of FIG. 1 and a drug delivery assembly for use with the spinal fixation device in accordance with another embodiment of the present disclosure;
FIG. 28 is a perspective view of the spinal fixation device of FIG. 1 and a drug delivery assembly for use with the spinal fixation device in accordance with an alternative embodiment of the present disclosure; and
FIG. 29 is a perspective view of the spinal fixation device of FIG. 23 and a shield for use with the spinal fixation device in accordance with an embodiment of the present disclosure, illustrating placement of the shield on the spinal fixation device in phantom.

### DETAILED DESCRIPTION OF THE EMBODIMENTS

Particular embodiments of the present disclosure will be described herein with reference to the accompanying drawings. As shown in the drawings and as described throughout the following description, and as is traditional when referring to relative positioning on an object, the terms "proximal" and "trailing" may be employed interchangeably, and should be understood as referring to the portion of a structure that is closer to a clinician during proper use. The terms "distal" and "leading" may also be employed interchangeably, and should be understood as referring to the portion of a structure that is farther from the clinician during proper use. In addition, the term "cephalad" or "cranial" is used in this application to indicate a direction toward a patient's head, whereas the term "caudad" indicates a direction toward the patient's feet. Further still, the term "medial" indicates a direction toward the middle of the body of the patient, whilst the term "lateral" indicates a direction toward a side of the body of the patient (i.e., away from the middle of the body of the patient). The term "posterior" indicates a direction toward the patient's back, and the term "anterior" indicates a direction toward the patient's front. In the following description, well-known functions or constructions are not described in detail to avoid obscuring the present disclosure in unnecessary detail.

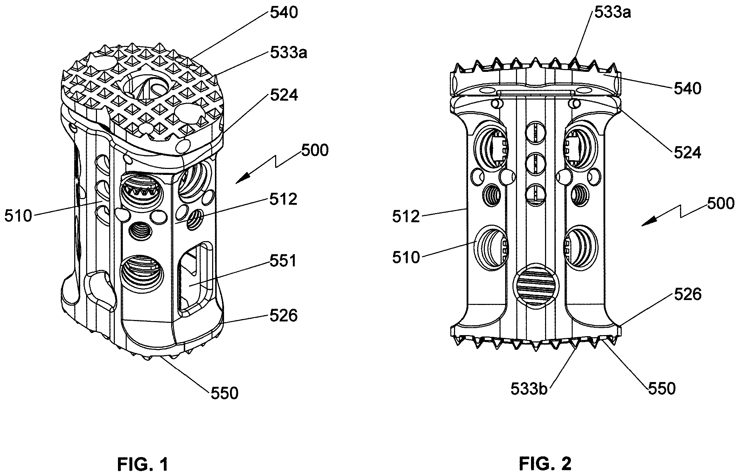

With reference to FIGS. 1-4 and 6, an embodiment of the present disclosure is shown generally as a spinal fixation device 500 configured and adapted to be positioned between vertebral bodies to support vertebral bodies and to promote spinal fusion. By way of example, spinal fixation device 500 may be inserted into the patient laterally, posteriorly, anteriorly, or obliquely. Additionally, spinal fixation device 500 may be inserted into the patient through procedures such as, e.g., posterior lumbar interbody fusion (PLIF), transforaminal lumbar interbody fusion (TLIF), lateral lumbar interbody fusion (LLIF), oblique lumbar interbody fusion (OLIF), or lateral extracavitary (LECA) procedures. Spinal fixation device 500 includes an outer housing 510 and an end plate assembly 560 interchangeably coupled with outer housing 510. Outer housing 510 includes a second end plate 550. End plate assembly 560 includes a first end plate 540, first and second elongate members 666, 668 operatively supporting first end plate 540, and a mounting assembly 600 operatively supporting first and second elongate members 666, 668. First and second end plates 540, 550 are configured to engage end plates of adjacent vertebral bodies. In particular, first and second end plates 540, 550 are configured to engage, e.g., endplates of superior and inferior vertebral bodies, respectively. Each of first and second end plates 540, 550 may include a plurality of pyramidal shaped spikes 533a, 533b (i.e., tetrahedrons) to aid in securing spinal fixation device 500 to the adjacent vertebral bodies for enhanced gripping of the vertebral bodies and minimizing movement of spinal fixation device 500 relative to the vertebral bodies. However, it is also contemplated that each of first and second end plates 540, 550 may include ridges or similar projections to aid in securing spinal fixation device 500 to the vertebral bodies.

End plate assembly 560 is configured as a modular assembly that is interchangeably mounted in outer housing 510. For example, a plurality of end plate assemblies 560 may be provided with varying parameters such as, e.g., footprint and lordosis, such that the clinician may selectively attach a desired end plate assembly 560 to outer housing 510 to meet the needs of each patient or surgical procedure being performed. In this manner, end plate assembly 560 may be tailored to achieve a desired lordosis of a first end plate 540 and a desired axial spacing between outer housing 510 and first end plate 540, as will be discussed hereinbelow.

Spinal fixation device 500 may be made of titanium, titanium alloy, stainless steel, allograft bone, autologous bone graft, polyetheretherketone (PEEK), cobalt chrome, polymeric materials, a combination thereof, or any other suitable biocompatible material. In particular, spinal fixation device 500 may be formed of bone, or an artificial material other than bone which may be harder or stronger than bone, such as, e.g., ceramic materials. Outer housing 510 may include a bone growth promoting material such as, e.g., bone morphogenic protein and hydroxyapatite. Outer housing 510 may define a cavity 551 to accommodate bone graft material therein.

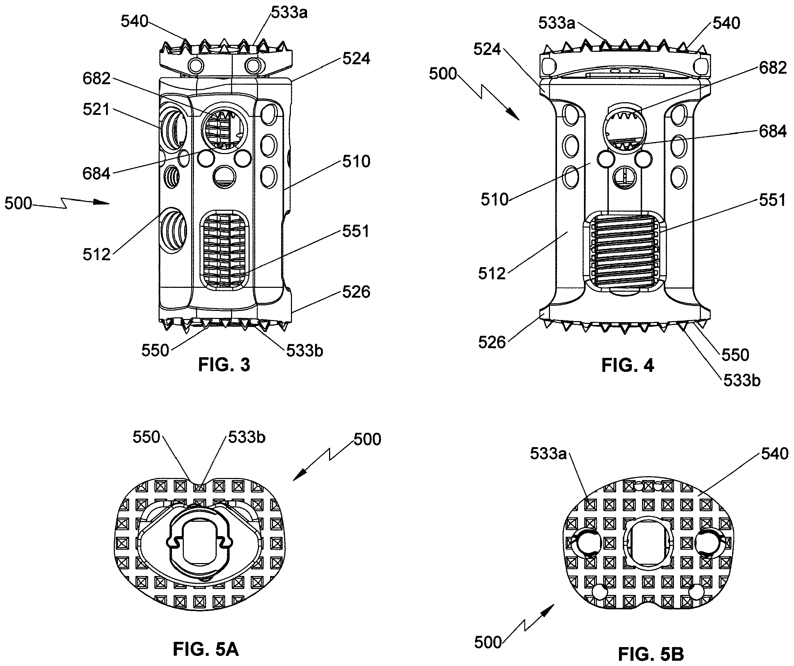

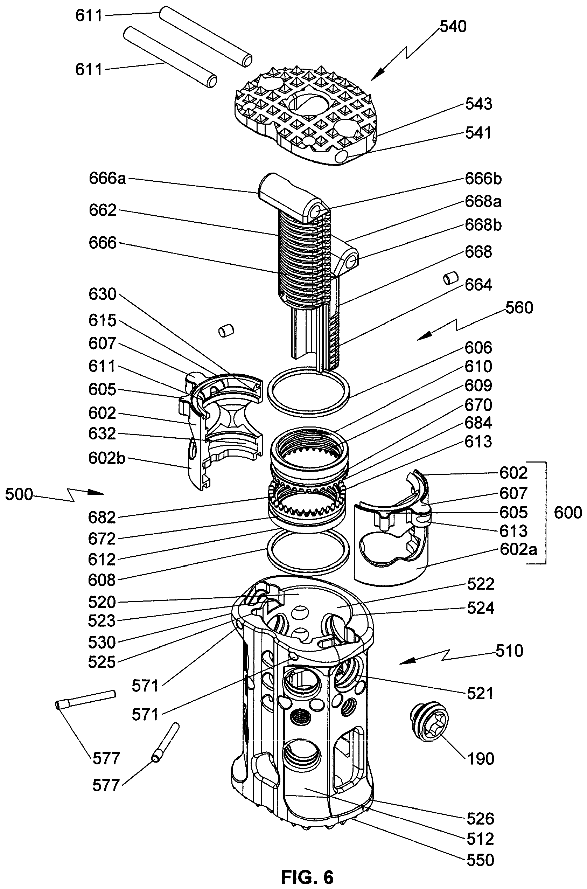

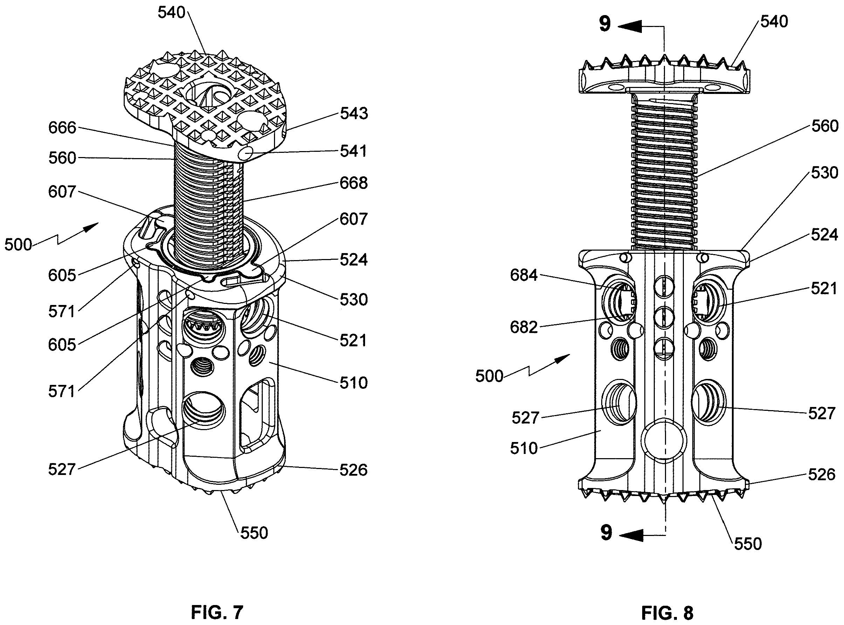

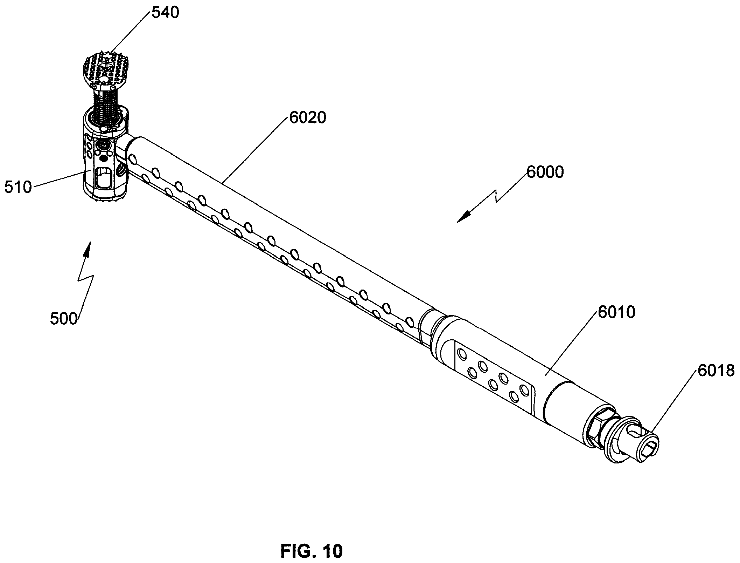

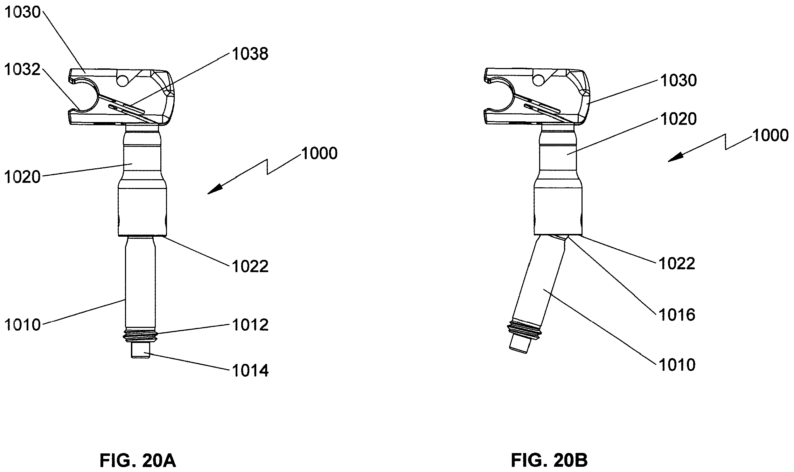

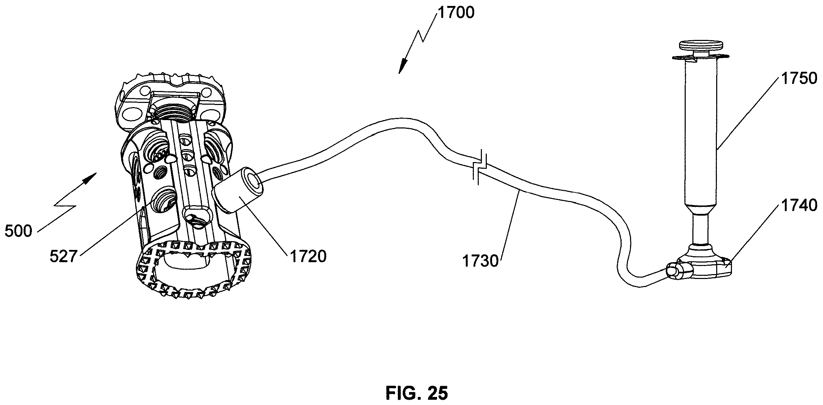

With reference to FIGS. 5A, 5B, and 6, outer housing 510 includes first and second ends 524, 526 and an outer wall 512 extending between first and second ends 524, 526. Outer wall 512 defines a plurality of bores 521 configured to receive a screw 190 and operatively engage insertion instrument 6000 (FIG. 10), as will be discussed hereinbelow. Bores 521 are circumferentially arranged about outer housing 510 to facilitate insertion of screw 190 and engagement with insertion instrument 6000 at different orientations. In addition, outer wall 512 further defines a plurality of bores 527 (FIGS. 7 and 8) circumferentially arranged about outer housing 510 to facilitate fixation of spinal stabilization devices such as, e.g., a rod connector 1000 (FIGS. 20a and 20b), or a drug delivery assembly 1700 (FIG. 25), as will be discussed hereinbelow. For example, the plurality of bores 527 may be defined in anterolateral portions of outer housing 510.

With particular reference to FIGS. 6-9, first end 524 of outer housing 510 defines an aperture 522, and second end 526 of outer housing 510 includes second end plate 550, e.g., integrally formed, with outer housing 510. Outer housing 510 defines a chamber 520 configured to receive at least a portion of end plate assembly 560 through aperture 522. End plate assembly 560 is selectively positionable within chamber 520. End plate assembly 560 includes a mounting assembly 600 releasably supported on a shoulder 530 of first end 524 of outer housing 510. Mounting assembly 600 includes a retaining housing 602 including first and second housing halves 602a, 602b, first and second rotatable members 610, 612 rotatably supported in retaining housing 602, and first and second retaining rings 606, 608.

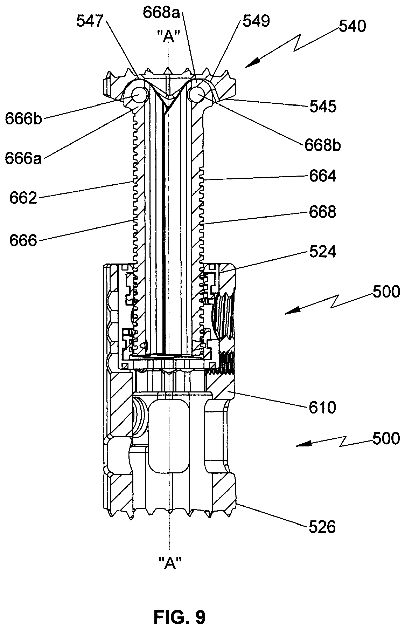

With particular reference to FIGS. 6 and 7, aperture 522 of first end 524 of outer housing 510 includes a non-circular cross-section complementary to a transverse cross-section of a first end 615 of retaining housing 602 to inhibit rotation of retaining housing 602 in aperture 522. In particular, each of first and second housing halves 602a, 602b includes radially extending first and second protrusions 605, 607 configured to be received in respective first and second recesses 523, 525 defined in shoulder 530 of outer housing 510. Outer housing 510 defines a pair of bores 571 transverse to a longitudinal axis "A" (FIG. 9) of spinal fixation device 500. Each bore 571 is configured to receive a spring pin 577. Each of first and second housing halves 602a, 602b defines a transverse groove 613 on respective second protrusions 607. Transverse groove 613 is configured to receive spring pin 577. Under such a configuration, spring pin 577 inserted through the respective bore 571 of outer housing 510 is received through transverse groove 613 of the respective second protrusion 607 to secure retaining housing 602 with outer housing 510. Spring pin 577 is flexible to enable the clinician to remove end plate assembly 560 from outer body 510 when end plate assembly 560 is pulled away from outer housing 510. When end plate assembly 560 is pulled away from outer housing 510, spring pin 577 flexes and disengages from transverse groove 613 of second protrusion 607. In this manner, the clinician is able to interchangeably utilize various end plate assemblies 560.

With continued reference to FIG. 6, each of first and second housing halves 602a, 602b includes an inner wall 611 defining first and second circumferential grooves 630, 632 configured to receive at least a portion of first and second retaining rings 606, 608, respectively. First rotatable member 610 defines a circumferential groove 670 configured to support first retaining ring 606 therein. First retaining ring 606 extends radially outward from circumferential groove 670 of first rotatable member 610 such that at least a portion of first retaining ring 606 is received in first circumferential groove 630 of retaining housing 602. In this manner, first rotatable member 610 is rotatably supported within retaining housing 602. Similarly, second rotatable member 612 defines a circumferential groove 672 configured to support second retaining ring 608 therein. Second retaining ring 608 extends radially outward from circumferential groove 672 such that at least a portion of second retaining ring 608 is received in second circumferential groove 632 of retaining housing 602. In this manner, second rotatable member 612 is rotatably supported within retaining housing 602. Under such a configuration, first and second rotatable members 610, 612 are rotatable with respect to retaining housing 602, while maintaining a fixed axial distance therebetween.

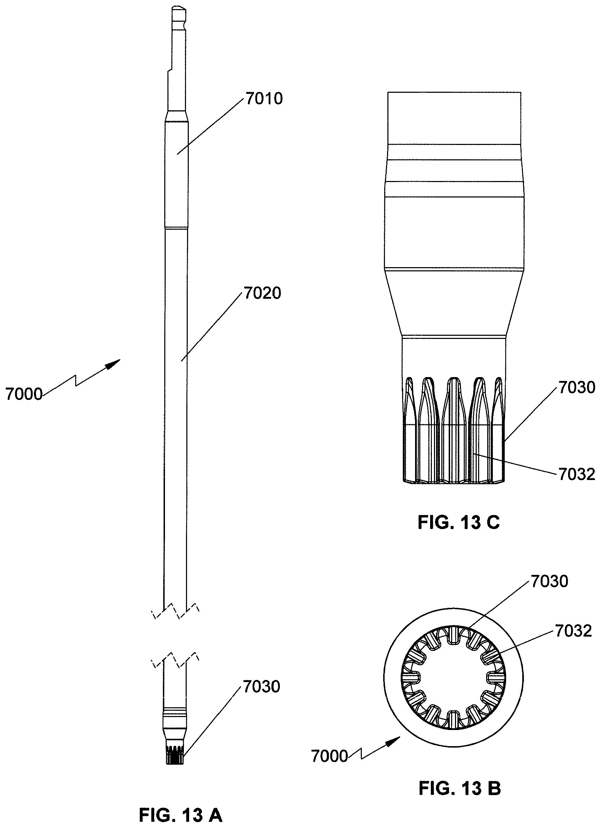

First and second rotatable members 610, 612 include internal threads 609, 613 (FIG. 6), respectively. In particular, internal threads 609, 613 are in opposite orientations, such that when first and second rotatable members 610, 612 rotate in opposite directions, the orientations of internal threads 609, 613 are the same. Internal threads 609, 613 are configured to threadably engage outer threads 662, 664 of first and second elongate members 666, 668 of end plate assembly 560, respectively. Under such a configuration, rotation of first and second rotatable members 610, 612 in opposite directions causes axial movement of first and second elongate members 666, 668 along longitudinal axis "A-A" (FIG. 9), as will be described hereinbelow. In addition, first and second rotatable members 610, 612 include opposing teeth 682, 684, respectively. Teeth 682, 684 are configured to engage, e.g., an engaging portion 7030 of a height adjusting driver 7000 (FIG. 13A), as will be discussed hereinbelow. Under such a configuration, rotation of height adjusting driver 7000 operatively coupled with teeth 682, 684 causes rotation of first and second rotatable members 610, 612, which, in turn, causes axial movement of first and second elongate members 666, 668.

With continued reference to FIGS. 7-9, end plate assembly 560 is selectively positionable relative to outer housing 510 through rotation of first and second rotatable members 610, 612 in opposite directions. In this manner, a length of spinal fixation device 500 may be selectively tailored to, e.g., the intervertebral space.

With particular reference now to FIG. 9, first end plate 540 includes an underside 545 defining first and second grooves 547, 549. First and second grooves 547, 549 are configured to receive round head portions 666a, 668a of first and second elongate members 666, 668, respectively. Round head portions 666a, 668a define respective bores 666b, 668b. First end plate 540 further defines bores 541, 543 (FIG. 7) configured to receive respective pins 611 (FIG. 6). In this manner, pins 611 pivotably secure round head portions 666a, 668a to first end plate 540. Under such a configuration, relative axial movement of first and second elongate members 666, 668 enables the clinician to adjust angular orientation of first end plate 540 with respect to longitudinal axis "A-A" (FIG. 9) to achieve the desired lordosis, as will be discussed hereinbelow.

First and second elongate members 666, 668 of end plate assembly 560 include outer threads 662, 664 configured to engage internal threads 609, 613 (FIG. 6) of first and second rotatable members 602, 404. First and second elongate members 666, 668 of end plate assembly 560 are movable relative to each other along longitudinal axis "A-A". In this manner, first end plate 540 may be advantageously angled to provide a desired amount of lordosis tailored to the need of each patient. For example, first end plate 540 may be positioned substantially orthogonal to the longitudinal axis "A-A" (FIG. 9) and adjacent first end 524 of outer housing 510. Alternatively, first end plate 540 may define an acute angle with longitudinal axis "A-A" (FIG. 9) and spaced apart from first end 524. The clinician may use angle adjusting driver 8000 to adjust the relative positioning of first and second elongate members 666, 668 to provide the adequate amount of lordosis of first end plate 540, as will be described hereinbelow.

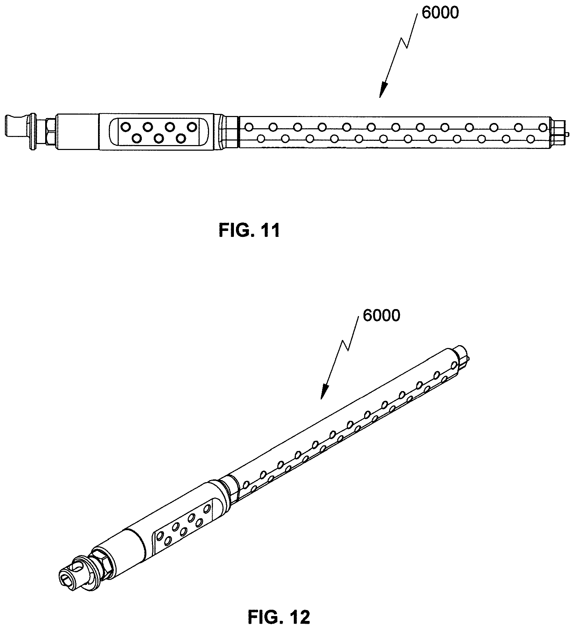

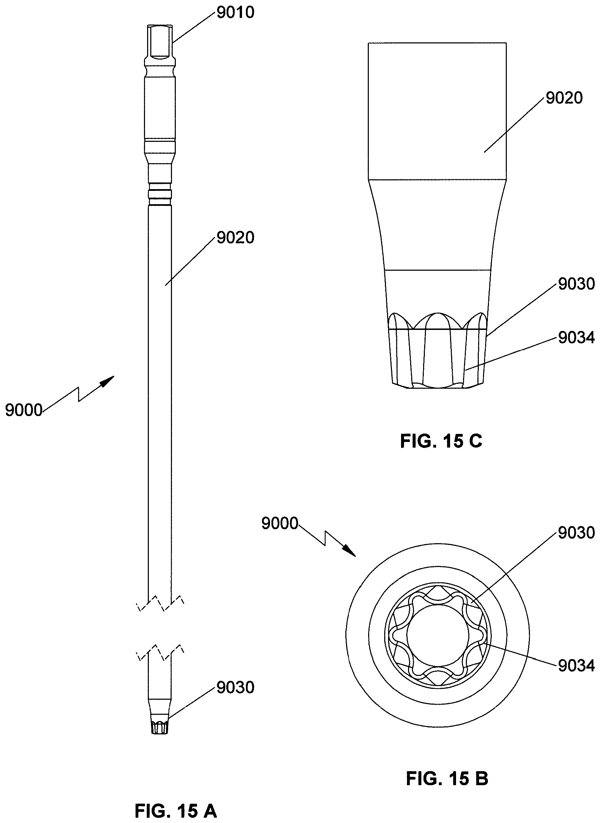

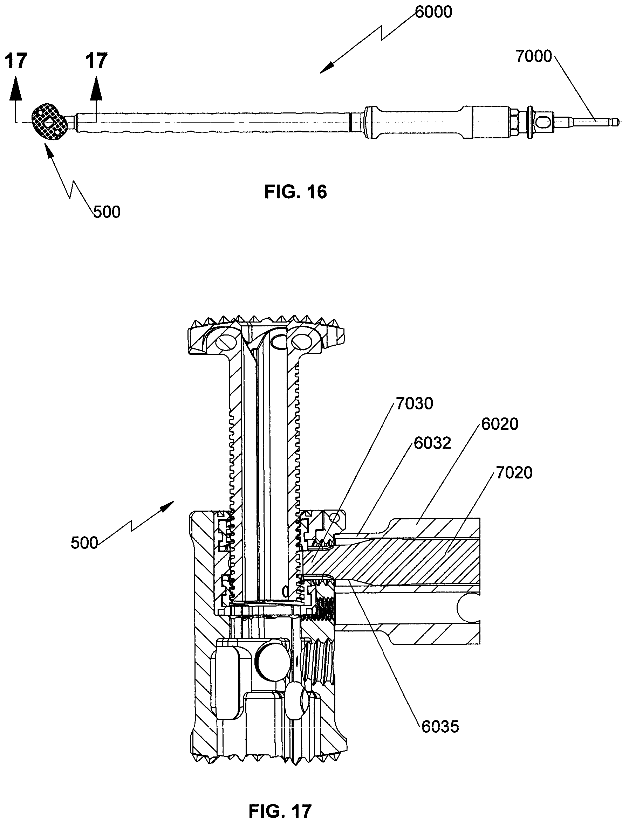

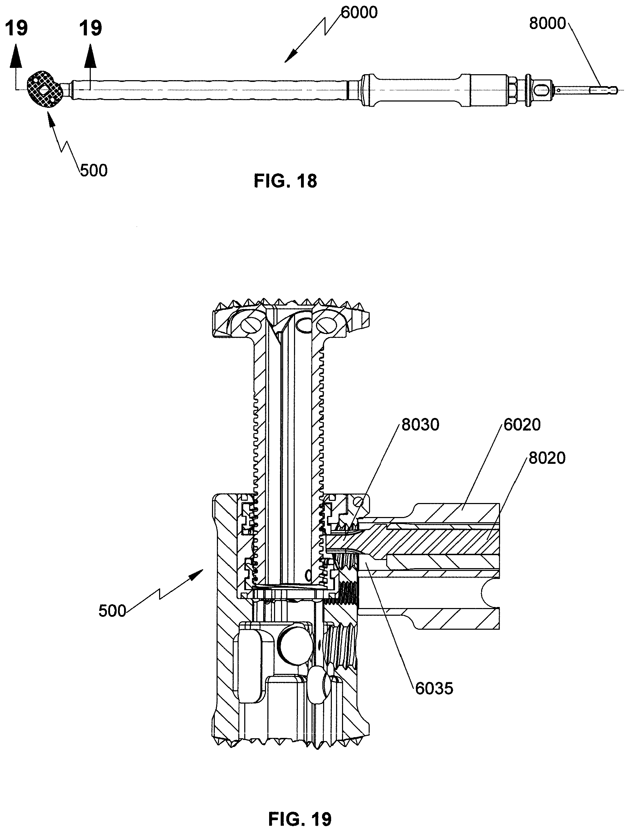

With reference now to FIGS. 10-12, there is shown an insertion instrument 6000 for use with spinal fixation device 500 to position spinal fixation device 500 between adjacent vertebral bodies. Insertion instrument 6000 includes a handle 6010 and an elongate body 6020 extending from handle 6010. Insertion instrument 6000 defines a channel 6035 (FIG. 17) configured to receive a height adjusting driver 7000 (a distal end of height adjusting driver 700 shown in FIG. 17), an angle adjusting driver 8000 (FIG. 18), or a tapered driver 9000 (FIGS. 15A-C) therethrough. Elongate body 6020 includes engaging portion 6032 (FIG. 17) configured to, e.g., threadably, engage bore 521 of outer housing 510 to securely attach spinal fixation device 500 with insertion instrument 6000.

With reference now to FIGS. 13A-C and FIG. 17, there is provided a height adjusting driver 7000 configured to be received through channel 6035 (FIG. 17) of insertion instrument 6000. Height adjusting driver 7000 includes a handle 7010, an elongate body 7020, and an engaging portion 7030. Engaging portion 7030 includes a plurality of teeth 7032 configured to engage teeth 684, 682 (FIG. 6) of first and second rotatable members 610, 612 of mounting assembly 600, respectively. In this manner, rotation of handle 7010 causes concomitant rotation of engaging portion 7030 about elongate body 7020, which, in turn, causes rotation of first and second rotatable members 610, 612 in opposite directions. Rotation of first and second rotatable members 610, 612 in opposite directions imparts axial translation of first and second elongate members 666, 668 as a single construct, which, in turn, enables the clinician to adjust the axial distance between first end plate 540 and outer housing 510, i.e., overall height of spinal fixation device 500. Rotation of handle 7010 in an opposite direction causes axial movement of end plate assembly 560 in an opposite direction.

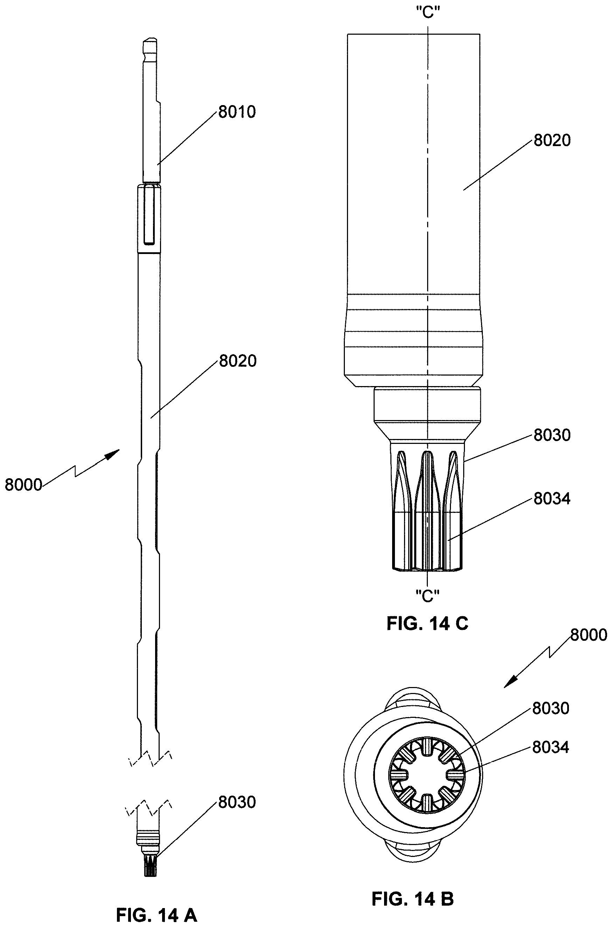

With reference now to FIGS. 14A-C and 19, there is provided an angle adjusting driver 8000 configured to be received through channel 6035 (FIG. 17) of insertion instrument 6000. Angle adjusting driver 8000 includes a handle 8010, an elongate body 8020, and an engaging portion 8030. For example, engaging portion 8030 may be radially offset from a central axis "C-C" defined by elongate body 8020. Engaging portion 8030 includes a plurality of teeth 8034 configured to engage teeth 682, 684 (FIG. 6) of either first or second rotatable members 610, 612 of mounting assembly 600. In this manner, rotation of handle 8010 causes rotation of one of the first or second rotatable members 610, 612 of mounting assembly 600. Rotation of only one rotatable member 610, 612 causes translation of one of first or second elongate member 666, 668. Relative movement between first and second elongate members 666, 668 changes angular orientation of first end plate 540 with respect to outer housing 510 and longitudinal axis "A-A." In this manner, the clinician may adjust the angular orientation of first end plate 540 to achieve the desired amount of lordosis or kyphosis. It is also contemplated that height adjusting driver 7000 and angle adjusting driver 8000 may be constructed as a single instrument that is configured to be received through channel 6035 (FIG. 17) of insertion instrument 6000.

With reference now to FIGS. 15A-C, there is provided a tapered driver 9000 configured to be received through channel 6035 (FIG. 17) of insertion instrument 6000. Tapered driver 9000 includes a handle 9010, an elongate body 9020, and an engaging portion 9030. Upon achieving the desired height of spinal fixation device 500 using height adjusting driver 7000 and angular orientation of first end plate 540 using angle adjusting driver 8000, screws 190 may be positioned in bore 521 of outer housing 510 through channel 6035 of insertion instrument 6000. Teeth 9034 of engaging portion 9030 engage teeth on a head portion of screw 190. Under such a configuration, handle 9010 may be rotated to threadably secure screw 190 in bore 521.

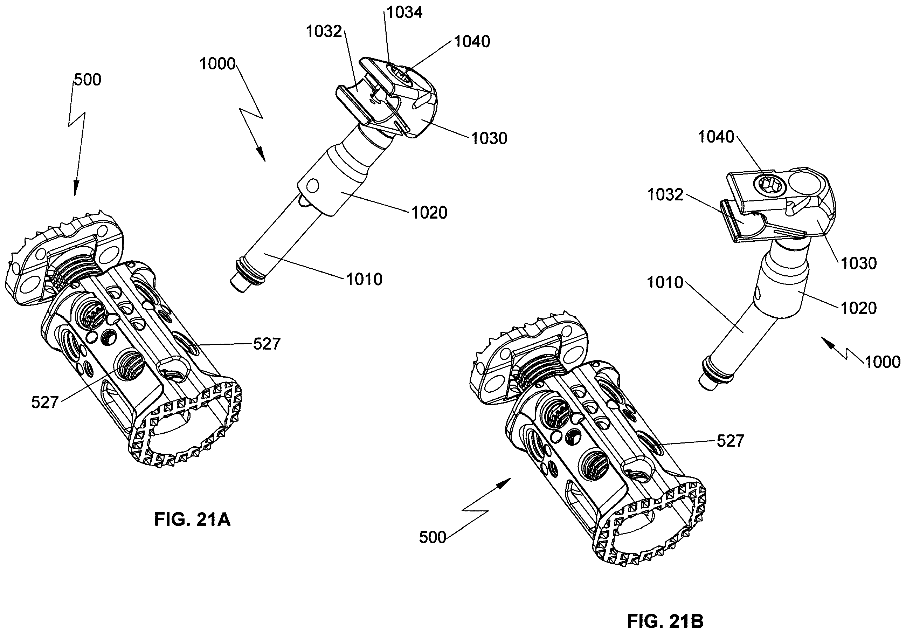

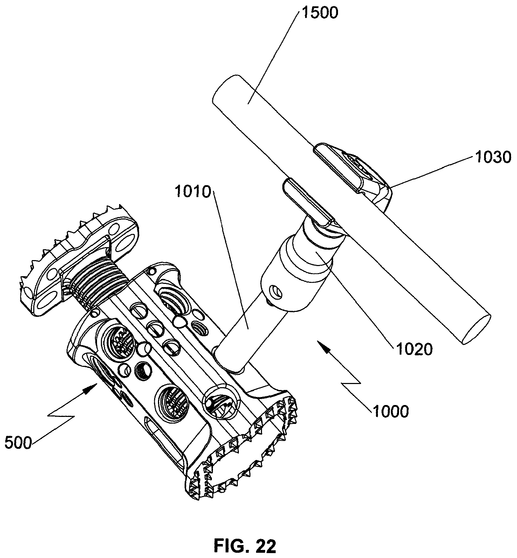

With reference now to FIGS. 20-21, a rod connector 1000 and a spinal rod 1500 (FIG. 22) may be used with spinal fixation device 500 to enhance securement of spinal fixation device 500 between vertebral bodies and to promote spinal fusion. Rod connector 1000 includes an anchoring portion 1010, a coupling portion 1020 rotatably and pivotably coupled with anchoring portion 1010, and a head portion 1030. Anchoring portion 1010 includes an engaging portion 1012 configured to be threadably coupled with bore 527 (FIGS. 21a and 21b) of spinal fixation device 500. Anchoring portion 1010 further includes a tip portion 1014 extending from engaging portion 1012. Tip portion 1014 has a diameter smaller than a diameter of engaging portion 1012 to facilitate alignment of engaging portion 1012 with bore 527. Anchoring portion 1010 further includes a ball joint 1016 (FIG. 20b). Coupling portion 1020 includes a socket 1022 configured to rotatably and pivotably receive ball joint 1016 of anchoring portion 1010. Under such a configuration, variable or polyaxial adjustments may be made between anchoring portion 1010 and coupling portion 1020. It is also contemplated that anchoring portion 1010 may include a socket and coupling portion 1020 may include a ball joint.

With reference to FIGS. 20-22, coupling portion 1020 is secured with head portion 1030. Head portion 1030 includes a recessed portion 1032 configured to receive spinal rod 1500 therethrough. Head portion 1030 defines slits 1038 configured to flex or enlarge the dimensions of recessed portion 1032 to facilitate insertion of spinal rod 1500. Head portion 1030 further defines a bore 1034 (FIG. 21a) configured to receive a screw 1040. Upon insertion of spinal rod 1500 in recessed portion 1032, screw 1040 may be fastened to secure spinal rod 1500 in recessed portion 1032. With particular reference to FIG. 22, spinal rod 1500 may be, e.g., a 5.5 mm diameter round rod. A reference may be made to U.S. Patent Application Serial No. 13/251,546, now U.S. Patent No. 8,920,471, filed on October 3, 2011, entitled "Transverse Connector," for a detailed discussion of the construction and operation of screws.

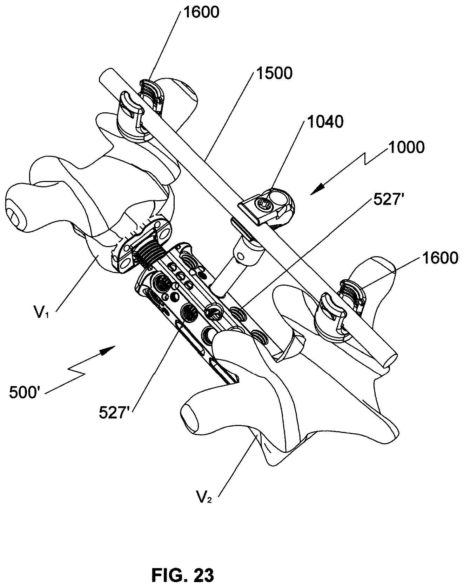

With reference now to FIG. 23, it is also envisioned that spinal fixation device 500 may be tailored to a particular surgical procedure being performed. For example, spinal fixation device 500' may include a plurality of bores 527' axially arranged along a length of spinal fixation device 500'. Under such a configuration, rod connector 1000 may be secured at a desired location along the length of spinal fixation device 500 to better secure spinal fixation device 500' between vertebral bodies v₁, v₂. In addition, it is also contemplated that additional rod connectors 1000 may be axially placed along the length of spinal fixation device 500' to further secure spinal fixation device 500'. In this manner, after spinal fixation device 500' is positioned between vertebral bodies v₁, v₂, pedicle screws 1600 may be secured to respective vertebral bodies v₁, v₂ and at least one rod connector 1000 may be secured with spinal fixation device 500'. Spinal rod 1500 may be received through the pedicle screws and rod connector 1000. Screw 1040 is utilized to secure spinal rod 1500 in recessed portion 1032 of rod connector 1000 and set screws (not shown) are used to secure spinal rod 1500 with pedicle screws 1600. A reference may be made to U.S. Patent Application Serial Nos. 12/739,461, now U.S. Patent No. 8,814,919, filed on August 26, 2014, entitled "Posterior Pedicle Screw Having a Taper Lock," and 12/739,506, filed on October 22, 2008, entitled "Polyaxial Screw Assembly," for a detailed discussion of the construction and operation of spinal rods and rod connectors.

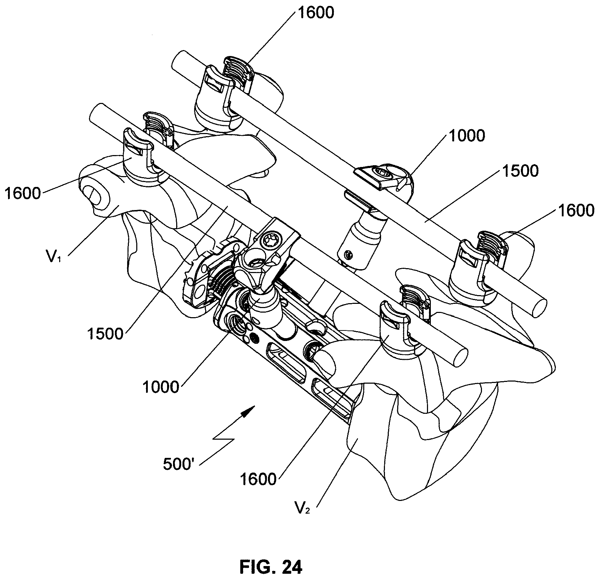

With reference to FIG. 24, it is also envisioned that a pair of spinal rods 1500 may be utilized to further secure spinal fixation device 500' between vertebral bodies v₁, v₂. As discussed hereinabove, additional rod connectors 1000 may be axially placed along the length of spinal fixation device 500'.

With reference now to FIG. 25, bores 527 may also be used to secure a drug delivery assembly 1700 to deliver a range of different synthetic or naturally occurring pharmaceutical or biological agents in liquid or gel formulations depending upon the particular application. Drugs may be administered for any actual or potential therapeutic, prophylactic or other medicinal purpose. Such drugs may include, e.g., analgesics, anesthetics, antimicrobial agents, antibodies, anticoagulants, antifibrinolytic agents, anti-inflammatory agents, antiparasitic agents, antiviral agents, cytokines, cytotoxins or cell proliferation inhibiting agents, chemotherapeutic agents, radiolabeled compounds or biologies, hormones, interferons, and combinations thereof. Thus, it is contemplated that spinal fixation device 500 may be used to deliver a formulation comprising an agent used in chemotherapy or radiotherapy.

Therapeutic agents may include chemotherapeutic agents (for example, paclitaxel, vincristine, ifosfamide, dacttinomycin, doxorubicin, cyclophosphamide, and the like), bisphosphonates (for example, alendronate, pamidronate, clodronate, zoledronic acid, and ibandronic acid), analgesics (such as opioids and NSAIDS), anesthetics (for example, ketoamine, bupivacaine and ropivacaine), tramadol, and dexamethasone. In other variations, drug delivery assembly 1700 may be used for delivering an agent useful in radiotherapy in, e.g., beads.

As an alternative to systemic administration of radioactive agents that are capable of targeting a particular tissue, antigen, or receptor type, the radioactive agents are administered locally following implantation of spinal fixation device 500. Such radiotherapy agents include radiolabeled antibodies, radiolabeled peptide receptor ligands, or any other radiolabeled compound capable of specifically binding to the specific targeted cancer cells.

In addition, drug delivery assembly 1700 may be used to deliver drugs used in the management of pain and swelling that occurs following the implantation surgery. For example, spinal fixation device 500 may release an effective amount of an analgesic agent alone or in combination with an anesthetic agent. As yet another alternative, spinal fixation device 500 may be used to deliver drugs which help minimize the risk of infection following implantation. For example, spinal fixation device 500 may release one or more antibiotics (for example, cefazolin, cephalosporin, tobramycin, gentamycin, etc.) and/or another agent effective in preventing or mitigating biofilms (for example, a quorum-sensing blocker or other agent targeting biofilm integrity). Bacteria may form biofilms on the surface of spinal fixation device 500, and these biofilms may be relatively impermeable to antibiotics. Accordingly, systemically administered antibiotics may not achieve optimal dosing where it is most needed. However, spinal fixation device 500 enables the delivery of the desired dose of antibiotic precisely when and where needed. In certain circumstances, the antibiotic may be delivered beneath the biofilm.

With reference to FIG. 25, drug delivery assembly 1700 includes a drug reservoir 1720 secured with bore 527, a catheter 1730 in fluid communication with drug reservoir 1720, and a supply port 1740 in fluid communication with catheter 1730. Supply port 1740 may be affixed to a location most convenient for the patient or the clinician to supply the drugs using, e.g., syringe 1750. For example, supply port 1740 may be affixed to an external location such as, e.g., the skin, of the patient. Supply port 1740 shown herein is merely exemplary and not drawn to scale. Supply port 1740 having different sizes, shapes, or profiles may be utilized for other applications such as, e.g., subcutaneous application.

The drugs discussed hereinabove, may be supplied through supply port 1740 by a syringe 1750. It is also contemplated that drug reservoir 1720 may be preloaded with a predetermined amount of drugs to eliminate catheter 1730, supply port 1740, and syringe 1750. It is further contemplated that spinal fixation device 500 may include channels, pores, and/or passages (not shown) formed in, e.g., outer housing 510. The channels, pores, and/or passages may be in fluid communication with drug reservoir 1720 to facilitate delivery of the drugs from drug reservoir 1720 to the delivery target.

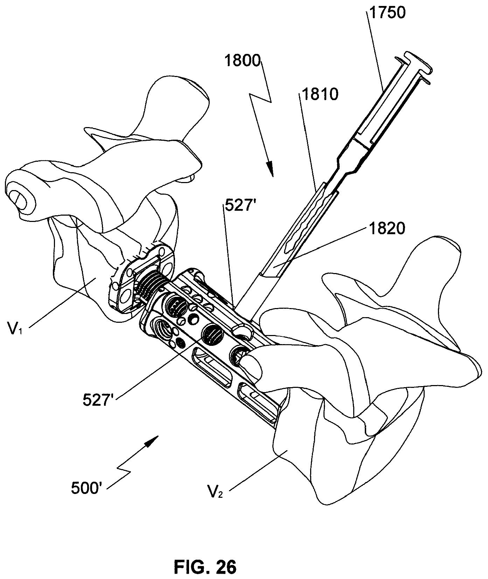

With reference to FIG. 26, bores 527' of spinal fixation device 500' or alternatively, bores 527 of spinal fixation device 500, may also be used to secure a drug delivery assembly 1800 to deliver the drugs. Drug delivery assembly 1800 includes a cannula 1810 defining a channel 1820 therethrough. The drugs discussed hereinabove, may be supplied to bores 527, 527' by syringe 1750 coupled to cannula 1810. It is contemplated that cannula 1810 may be coupled to spinal fixation device 500, 500' to supply the drugs to the delivery target during the surgical procedure and may be removed after the completion of the surgical procedure.

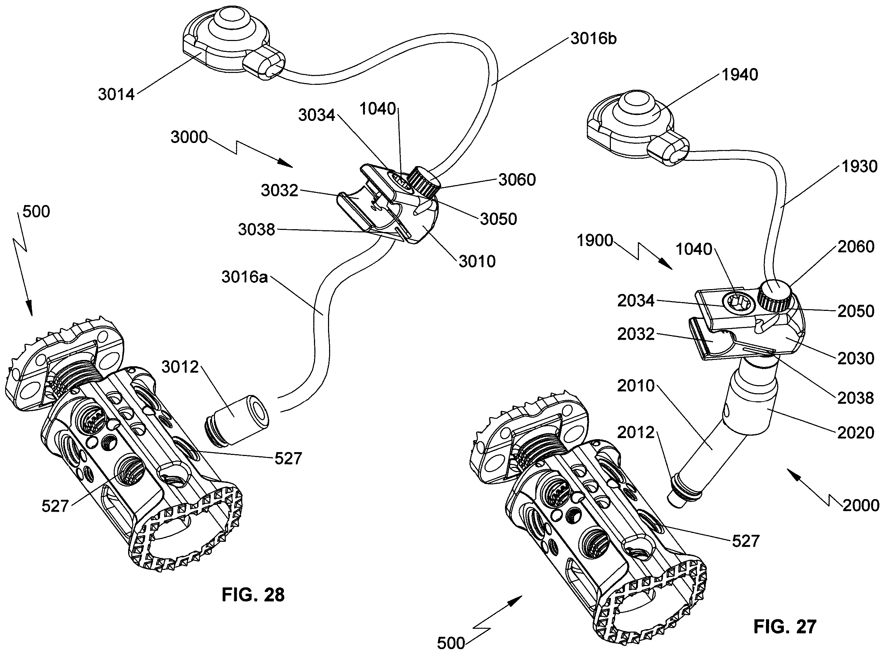

With reference now to FIG. 27, it is also envisioned that a drug delivery assembly 1900 may be utilized to deliver the drugs to the delivery target. Drug delivery assembly 1900 includes rod connector 2000, a seal member 2060, a supply port 1940, and a catheter 1930 interconnecting supply port 1940 and rod connector 2000. In particular, rod connector 2000 defines a channel (not shown) extending therethrough such that when rod connector 2000 is coupled with spinal fixation device 500, 500', the channel is in fluid communication with bore 527, 527' of spinal fixation device 500, 500' to facilitate drug delivery through rod connector 2000.

Rod connector 2000 has a substantially similar configuration as rod connector 1000. The portions of rod connector 2000 that are identical to rod connector 1000, as discussed hereinabove, will not be discussed in detail. Rod connector 2000 includes an anchoring portion 2010, a coupling portion 2020 rotatably and pivotably coupled with anchoring portion 2010, and a head portion 2030. Anchoring portion 2010 includes an engaging portion 2012 configured to be threadably coupled with bore 527 of spinal fixation device 500. Coupling portion 2020 is secured with head portion 2030. Head portion 2030 includes a recessed portion 2032 configured to receive spinal rod 1500 therethrough. Head portion 2030 defines slits 2038 configured to flex or enlarge the dimensions of recessed portion 2032 to facilitate insertion of spinal rod 1500. Head portion 1030 further defines a bore 2034 configured to receive screw 1040. Upon insertion of spinal rod 1500 in recessed portion 2032, screw 1040 may be fastened to secure spinal rod 1500 in recessed portion 2032. Furthermore, head portion 2030 defines a bore 2050 in communication with the channel. Bore 2050 is dimensioned to receive seal member 2060 in a sealing relation. Under such a configuration, when the patient or the clinician supplies the drugs through supply port 1940 using, e.g., syringe 1750 (FIG. 25), the drugs flow through the channel of rod connector 2000 and to the drug delivery target through bore 527, 527' of spinal fixation device 500, 500'. Alternatively, rod connector 2000 may include a drug reservoir preloaded with a predetermined amount of drugs such that catheter 1930, supply port 1940, and seal member 2060 may be eliminated. Under such a configuration, a plug may be used to close bore 2050 in communication with the channel. In this manner, rod connector 2000 may provide, e.g., time-release, drug delivery.

With reference now to FIG. 28, it is also envisioned that a drug delivery assembly 3000 may be utilized to deliver the drugs to the delivery target. In particular, drug delivery assembly 3000 includes a rod connector 3010, an engaging portion 3012, a seal member 3060, a supply port 3014, and catheters 3016a, 3016b. Rod connector 3010 is substantially identical to head portion 2030 of drug delivery assembly 1900, and thus, will not be described in detail herein. Rod connector 3010 includes a recessed portion 3032 configured to receive spinal rod 1500 therethrough. Rod connector 3010 defines slits 3038 configured to flex or enlarge the dimensions of recessed portion 3032 to facilitate insertion of spinal rod 1500. Rod connector 3010 further defines a bore 3034 configured to receive screw 1040. Upon insertion of spinal rod 1500 in recessed portion 3032, screw 1040 may be fastened to secure spinal rod 1500 in recessed portion 3032. Furthermore, connecting rod 3010 defines a bore 3050. Bore 3050 is dimensioned to receive seal member 3060 in a sealing relation. Catheter 3016a connects bore 3050 with engaging portion 3012, and catheter 3016b interconnects supply port 3014 with seal member 3060. Engaging portion 3012 may be e.g., threadably, coupled with bore 527, 527' of spinal fixation device 500, 500'. Under such a configuration, when the patient or the clinician supplies the drugs to supply port 3014 using, e.g., syringe 1750 (FIG. 25), the drugs flow through rod connector 3010 and bores 527, 527' of spinal fixation device 500, 500' to the drug delivery target. Alternatively, rod connector 3010 may include a drug reservoir preloaded with a predetermined amount of drugs such that catheter 3016b, supply port 3014, and seal member 3060 are eliminated. Under such a configuration, a plug (not shown) may be used to close bore 3050. In this manner, rod connector 3010 may provide, e.g., time-release, drug delivery.

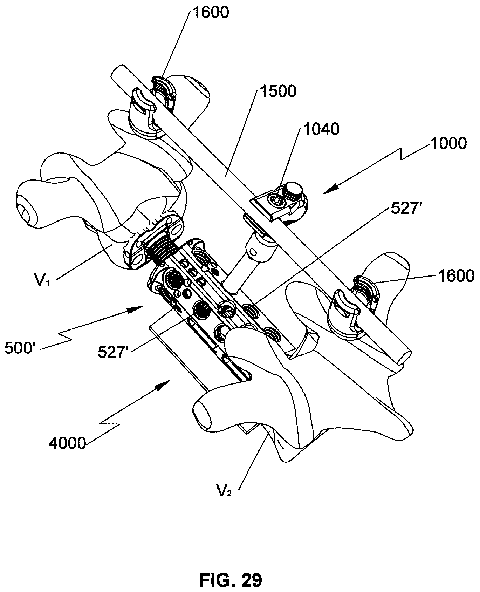

With reference to FIG. 29, a shield for use with spinal fixation device 500, 500' is generally shown as 4000. Shield 4000 may be affixed to spinal fixation device 500, 500' using adhesive, ultrasonic welding or other suitable means. Shield 4000 may include, e.g., polyphenylene sulfide (PPS), to reduce radiation to the spinal cord during, e.g., post-operative radiotherapy. In addition, shield 4000 may serve as a barrier for bone cement such as, e.g., polymethyl methacrylate (PMMA), drugs, or bone graft products and biologics. In addition, shield 4000 may include, e.g., slow release agents such as antibiotics.

In use, the clinician first distracts vertebral bodies of interest to establish the intervertebral space. The clinician may then remove vertebral tissue, if necessary or desired. First and second elongate members 666, 668 of end plate assembly 560 are selectively positioned to achieve a desired orientation of first end plate 540. Insertion instrument 6000 is coupled with spinal fixation device 500 by, e.g., threadably, coupling engaging portion 6032 (FIG. 17) with bore 521. Spinal fixation device 500 is then positioned adjacent a desired intervertebral space between vertebral bodies. Upon inserting spinal fixation device 500 in the intervertebral space, height adjusting driver 7000 can be inserted through channel 6035 (FIGS. 16 and 17) of insertion instrument 6000 to further adjust the axial distance between first end plate 560 and outer housing 510 by placing engaging portion 7030 through one of the plurality of bores 521 defined in outer housing 510 such that teeth 7032 of engaging portion 7030 of height adjusting driver 7000 engages teeth 684, 682 of first and second rotatable members 610, 612 of mounting assembly 600, respectively. In this manner, rotation of handle 7010 causes rotation of first and second rotatable members 610, 612 in opposite directions, which, in turn, imparts axial translation of first and second elongate members 666, 668. In this manner, the clinician may adjust the axial distance between first end plate 540 and outer housing 510, i.e., height of spinal fixation device 500. Handle 7010 is rotated until a desired height of spinal fixation device 500 is effected through axial movement of end plate assembly 560.

In addition, after removing height adjusting driver 7000 from insertion instrument 6000, angle adjusting driver 8000 can be inserted through channel 6035 (FIG. 19) of insertion instrument 6000 to further adjust the angular orientation of first end plate 540 with respect to outer housing 510. Engaging portion 8030 is inserted through one of the plurality of bores 521 defined in outer housing 510 such that teeth 8034 of engaging portion 8030 of angle adjusting driver 8000 engages teeth 682 of first rotatable member 610 or teeth 684 of second rotatable member 612 of mounting assembly 600. Rotation of handle 8010 causes rotation of one of the first or second rotatable members 610, 612 of mounting assembly 600, which, in turn, causes translation of one of first or second elongate members 666, 668. Relative movement between first and second elongate members 666, 668 enables the clinician to adjust the angular orientation of first end plate 540 with respect to outer housing 510 to achieve the desired lordosis or kyphosis. It is contemplated that the clinician may make further adjustments by alternating height adjusting driver 7000 and angle adjusting driver 8000 to achieve the desired length of spinal fixation device 500 and angular orientation of first end plate 540. Upon achieving the desired length of spinal fixation device 500 and angular orientation of first end plate 540, screw 190 may be placed in bore 521 defined in outer housing 510 through channel 6035 of insertion instrument 6000. Screw 190 may be threadably secured in bore 521 by using tapered driver 9000.

At this time, rod connector 1000, pedicle screws 1600, and spinal rod 1500 may be utilized to further secure spinal fixation device 500 between vertebral bodies and to further promote spinal fusion. Anchoring portion 1010 of rod connector 1000 is threadably coupled with bore 527 of spinal fixation device 500, and pedicle screws 1600 are secured with respective vertebral bodies. Thereafter, spinal rod 1500 is inserted through recessed portion 1032 of rod connector 1000 and respective pedicle screws 1600. At this time, rod connector 1000 may be adjusted for proper angular orientation. Screw 1040 may be used to secure spinal rod 1500 in recessed portion 1032 of rod connector 1000 and the set screws (not shown) may be used to secure spinal rod 1500 with pedicle screws 1600. At this time, additional rod connector 1000 may be used to further secure spinal fixation device 500 between vertebral bodies. At this time, additional spinal rod 1500 may be utilized to further secure spinal fixation device 500 (FIG. 24).

Based on the needs of the patient, drug delivery assemblies 1700, 1800, 1900, 3000 may be utilized to deliver the necessary drugs to the patient. To this end, drug reservoir 1720 of drug delivery assembly 1700 is secured with bore 527 of spinal fixation device 500. Supply port 1740 may be affixed to a location most convenient for the patient or the clinician to supply the drugs using, e.g., syringe 1750. Alternatively, cannula 1810 of drug delivery assembly 1800 is secured with bore 527 of spinal fixation device 500. The patient or the clinician may administer the drugs using syringe 1750. The use of drug delivery assemblies 1900, 2000 is substantially identical to the use of drug delivery assemblies 1700, 1800, and thus, will not be described herein. It is also contemplated that a drug pump such as, e.g., an insulin pump, may be connected to catheter 1730 to supply the drugs.

## Claims

1. A spinal fixation device (500) comprising:
an outer housing (510) defining an aperture (522) and a longitudinal axis; and
an end plate assembly (560) coupled with the outer housing, at least a portion of the end plate assembly slidably received within the outer housing (510) through said aperture of the outer housing, (510), the end plate assembly (560) including a first end plate (540) configured to engage a vertebral body, wherein the end plate assembly (560) is selectively movable between a first position in which the first end plate (540) is spaced apart from the outer housing (510) and a second position in which the first end plate (540) is adjacent the outer housing (510), and the first end plate (540) is selectively adjustable to an angular orientation of a plurality of angular orientations with respect to the longitudinal axis of the outer housing (510),
wherein the end plate assembly (560) includes first and second elongate members (666, 668) operatively coupled to the first end plate (540) such that axial movement of the first and second elongate members (666, 668) in tandem causes axial displacement of the first end plate (540), and relative movement between the first and second elongate members (666, 668) transitions the first end plate (540) from a first angular orientation to a second angular orientation, and wherein the spinal fixation device further comprises a mounting assembly (600) operatively supporting the first and second elongate members (666, 668) in the outer housing (510),
**characterized in that** the mounting assembly (600) includes a retaining housing (602) and first and second rotatable members (610, 612) rotatably supported in the retaining housing (602), the first and second rotatable members (610, 612) operatively coupled with the first and second elongate members (666, 668) of the end plate assembly (560) respectively.

2. The spinal fixation device according to claim 1, wherein the mounting assembly (600) is releasably secured with the outer housing (510).

3. The spinal fixation device according to claim 1 or 2, wherein the first and second rotatable members (610, 612) of the mounting assembly (600) are spaced apart from each other to define a gap therebetween.

4. The spinal fixation device according to claim 1, 2 or 3, wherein each of the first and second rotatable members (610, 612) of the mounting assembly (600) includes circumferentially arranged teeth opposing each other.

5. The spinal fixation device according to any preceding claim, wherein the first rotatable member (610) of the mounting assembly (600) includes inner threads in a first orientation and the second rotatable member (612) of the mounting assembly (600) includes inner threads in a second orientation opposite to the first orientation.

6. The spinal fixation device according to claim 3, 4 or 5, wherein the outer housing (510) defines a plurality of bores, at least one bore of the plurality of bores in communication with the gap defined between the first and second rotatable members (610, 612) of the mounting assembly (600).

7. The spinal fixation device according to claim 6, wherein the plurality of bores are circumferentially arranged about the outer housing (510), or
wherein the plurality of bores are arranged along a length of the outer housing (510).

8. A kit for spinal surgery comprising:
a spinal fixation device (500) according to any preceding claim; and
a surgical instrument defining a channel extending therethrough, the surgical instrument defining an engaging portion configured to securely engage the outer housing of the spinal fixation device.

9. The kit for spinal surgery according to claim 8 when dependent on claim 4, wherein the surgical instrument includes a height adjusting driver (7000) rotatably extending through the channel of the surgical instrument, the height adjusting driver (7000) including an engaging portion having teeth configured to engage the circumferentially arranged teeth of the first and second rotatable members (610, 612) of the mounting assembly (600), whereby rotation of the height adjusting driver (7000) causes rotation of the first and second rotatable members (610, 612) of the mounting assembly (600), and/or
wherein the surgical instrument further includes an angle adjusting driver (8000) rotatably extending through the channel of the surgical instrument, the angle adjusting driver (8000) including an engaging portion having teeth configured to engage the circumferentially arranged teeth of one of the first or second rotatable members (610, 612) of the mounting assembly (600), whereby rotation of the angle adjusting driver (8000) causes rotation of the one of the first or second rotatable members (610, 612) of the mounting assembly (600).

10. The kit for spinal surgery according to claim 8 or 9, comprising a rod connector (1000) in addition to or instead of the surgical instrument, the rod connector (1000) having an anchoring portion coupled with the outer housing of the spinal fixation device (500) and a head portion having a recessed portion configured to receive a spinal rod, preferably
wherein the rod connector (1000) defines a channel therethrough, more preferably
wherein the head portion of the rod connector (1000) defines a bore in communication with the channel.

11. The kit for spinal surgery according to claim 8, 9 or 10, further comprising a drug delivery assembly in addition to or instead of the surgical instrument, the drug delivery assembly including a drug reservoir securely coupled with the outer housing (510) of the spinal fixation device (500), a supply port, and a catheter in fluid communication with the supply port and the drug reservoir.

12. The kit for spinal surgery according to claim 8, 9, 10 or 11, further comprising a shield (4000) in addition to or instead of the surgical instrument, the shield attachable to the spinal fixation device (500), preferably
wherein the shield (4000) is configured to reduce radiation to a spinal cord, more preferably
wherein the shield (4000) includes slow releasing medicament.

## Patentansprüche

1. Wirbelsäulenfixierungsvorrichtung (500), umfassend:
ein äußeres Gehäuse (510), das eine Öffnung (522) und eine Längsachse definiert; und
eine Endplattenbaugruppe (560), die mit dem äußeren Gehäuse gekoppelt ist, wobei mindestens ein Abschnitt der Endplattenbaugruppe durch die Öffnung des äußeren Gehäuses hindurch verschiebbar innerhalb des äußeren Gehäuses (510) aufgenommen ist,
wobei die Endplattenbaugruppe (560) eine erste Endplatte (540), konfiguriert zum Eingriff mit einem Wirbelkörper, einschließt, wobei die Endplattenbaugruppe (560) selektiv zwischen einer ersten Position, in der die erste Endplatte (540) vom äußeren Gehäuse (510) beabstandet ist, und einer zweiten Position beweglich ist, in der die erste Endplatte (540) an das äußere Gehäuse (510) angrenzt, und die erste Endplatte (540) selektiv auf eine Winkelausrichtung einer Vielzahl von Winkelausrichtungen in Bezug auf die Längsachse des äußeren Gehäuses (510) einstellbar ist,
wobei die Endplattenbaugruppe (560) erste und zweite längliche Elemente (666, 668) einschließt, die betriebsfähig derart mit der ersten Endplatte (540) gekoppelt sind, dass axiale Tandembewegung des ersten und zweiten länglichen Elements (666, 668) axiale Verlagerung der ersten Endplatte (540) bewirkt, und Relativbewegung zwischen dem ersten und zweiten länglichen Element (666, 668) die erste Endplatte (540) von einer ersten Winkelausrichtung zu einer zweiten Winkelausrichtung umstellt, und wobei die Wirbelsäulenfixierungsvorrichtung weiter eine Montagebaugruppe (600) umfasst, die das erste und zweite längliche Element (666, 668) betriebsfähig im äußeren Gehäuse (510) abstützt,
**dadurch gekennzeichnet, dass** die Montagebaugruppe (600) ein Haltegehäuse (602) und erste und zweite drehbare Elemente (610, 612) einschließt, die drehbar im Haltegehäuse (602) abgestützt sind, wobei das erste und zweite drehbare Element (610, 612) betriebsfähig mit jeweils dem ersten und zweiten länglichen Element (666, 668) der Endplattenbaugruppe (560) gekoppelt sind.

2. Wirbelsäulenfixierungsvorrichtung nach Anspruch 1, wobei die Montagebaugruppe (600) lösbar am äußeren Gehäuse (510) gesichert ist.

3. Wirbelsäulenfixierungsvorrichtung nach Anspruch 1 oder 2, wobei das erste und zweite drehbare Element (610, 612) der Montagebaugruppe (600) voneinander beabstandet sind, um einen Spalt dazwischen zu definieren.

4. Wirbelsäulenfixierungsvorrichtung nach Anspruch 1, 2 oder 3, wobei jedes von dem ersten und zweiten drehbaren Element (610, 612) der Montagebaugruppe (600) umfangsmäßig angeordnete Zähne einschließt, die einander gegenüberliegen.

5. Wirbelsäulenfixierungsvorrichtung nach einem vorstehenden Anspruch, wobei das erste drehbare Element (610) der Montagebaugruppe (600) Innengewinde in einer ersten Ausrichtung einschließt, und das zweite drehbare Element (612) der Montagebaugruppe (600) Innengewinde in einer zweiten Ausrichtung einschließt, die der ersten Ausrichtung entgegengesetzt ist.

6. Wirbelsäulenfixierungsvorrichtung nach Anspruch 3, 4 oder 5, wobei das äußere Gehäuse (510) eine Vielzahl von Bohrungen definiert, mindestens eine Bohrung der Vielzahl von Bohrungen in Kommunikation mit dem Spalt, der zwischen dem ersten und zweiten drehbaren Element (610, 612) der Montagebaugruppe (600) definiert ist.

7. Wirbelsäulenfixierungsvorrichtung nach Anspruch 6, wobei die Vielzahl von Bohrungen umfangsmäßig um das äußere Gehäuse (510) herum angeordnet sind, oder
wobei die Vielzahl von Bohrungen entlang einer Länge des äußeren Gehäuses (510) angeordnet sind.

8. Wirbelsäulenchirurgie-Kit, umfassend:
eine Wirbelsäulenfixierungsvorrichtung (500) nach einem der vorstehenden Ansprüche; und
ein chirurgisches Instrument, das einen Kanal definiert, der sich dort hindurch erstreckt, wobei das chirurgische Instrument einen Eingriffsabschnitt definiert, konfiguriert, um sicher mit dem äußeren Gehäuse der Wirbelsäulenfixierungsvorrichtung einzugreifen.

9. Wirbelsäulenchirurgie-Kit nach Anspruch 8 wenn abhängig von Anspruch 4, wobei das chirurgische Instrument einen Höheneinstellungsdrehereinsatz (7000) einschließt, der sich drehbar durch den Kanal des chirurgischen Instruments hindurch erstreckt, wobei der Höheneinstellungsdrehereinsatz (7000) einen Eingriffsabschnitt einschließt, der Zähne aufweist, konfiguriert, um mit den umfangsmäßig angeordneten Zähnen des ersten und zweiten drehbaren Elements (610, 612) der Montagebaugruppe (600) einzugreifen, wodurch Drehung des Höheneinstellungsdrehereinsatzes (7000) Drehung des ersten und zweiten drehbaren Elements (610, 612) der Montagebaugruppe (600) bewirkt, und/oder
wobei das chirurgische Instrument weiter einen Winkeleinstellungsdrehereinsatz (8000) einschließt, der sich drehbar durch den Kanal des chirurgischen Instruments hindurch erstreckt, wobei der Winkeleinstellungsdrehereinsatz (8000) einen Eingriffsabschnitt einschließt, der Zähne aufweist, konfiguriert, um mit den umfangsmäßig angeordneten Zähnen von einem aus dem ersten oder zweiten drehbaren Element (610, 612) der Montagebaugruppe (600) einzugreifen, wodurch Drehung des Winkeleinstellungsdrehereinsatzes (8000) Drehung des einen von dem ersten oder zweiten drehbaren Element (610, 612) der Montagebaugruppe (600) bewirkt.

10. Wirbelsäulenchirurgie-Kit nach Anspruch 8 oder 9, das zusätzlich zu oder anstelle des chirurgischen Instruments einen Stabverbinder (1000) umfasst, wobei der Stabverbinder (1000) einen Verankerungsabschnitt aufweist, der mit dem äußeren Gehäuse der Wirbelsäulenfixierungsvorrichtung (500) gekoppelt ist, und einen Kopfabschnitt mit einem vertieften Abschnitt, konfiguriert, um einen Wirbelsäulenstab aufzunehmen, vorzugsweise
wobei der Stabverbinder (1000) einen Kanal dort hindurch definiert, mehr bevorzugt
wobei der Kopfabschnitt des Stabverbinders (1000) eine Bohrung in Kommunikation mit dem Kanal definiert.

11. Wirbelsäulenchirurgie-Kit nach Anspruch 8, 9 oder 10, das weiter zusätzlich zu oder anstelle des chirurgischen Instruments eine Arzneimittelabgabebaugruppe umfasst, wobei die Arzneimittelabgabebaugruppe ein sicher mit dem äußeren Gehäuse (510) der Wirbelsäulenfixierungsvorrichtung (500) gekoppeltes Arzneimittelreservoir, einen Zuführanschluss, und einen Katheter in Fluidkommunikation mit dem Zuführanschluss und dem Arzneimittelreservoir einschließt.

12. Wirbelsäulenchirurgie-Kit nach Anspruch 8, 9, 10 oder 11, das weiter zusätzlich zu oder anstelle des chirurgischen Instruments eine Abschirmung (4000) umfasst, wobei die Abschirmung an der Wirbelsäulenfixierungsvorrichtung (500) anbringbar ist, vorzugsweise
wobei die Abschirmung (4000) konfiguriert ist, um Strahlung zu einem Rückenmark zu verringern, mehr bevorzugt
wobei die Abschirmung (4000) langsam freisetzendes Medikament einschließt.

## Revendications

1. Dispositif de fixation spinale (500) comprenant :
un boîtier extérieur (510) définissant une ouverture (522) et un axe longitudinal ; et
un ensemble de plaque d'extrémité (560) accouplé au boîtier extérieur, au moins une partie de l'ensemble de plaque d'extrémité étant reçue de manière coulissante dans le boîtier extérieur (510) à travers ladite ouverture du boîtier extérieur, l'ensemble de plaque d'extrémité (560) incluant une première plaque d'extrémité (540) configurée pour venir en prise avec un corps vertébral, dans lequel l'ensemble de plaque d'extrémité (560) est mobile sélectivement entre une première position dans laquelle la première plaque d'extrémité (540) est espacée du boîtier extérieur (510) et une seconde position dans laquelle la première plaque d'extrémité (540) est adjacente au boîtier extérieur (510), et la première plaque d'extrémité (540) est réglable sélectivement selon une orientation angulaire d'une pluralité d'orientations angulaires par rapport à l'axe longitudinal du boîtier extérieur (510),
dans lequel l'ensemble de plaque d'extrémité (560) inclut des premier et second éléments allongés (666, 668) accouplés de manière opérationnelle à la première plaque d'extrémité (540) de sorte qu'un mouvement axial des premier et second éléments allongés (666, 668) en tandem entraîne un déplacement axial de la première plaque d'extrémité (540), et qu'un mouvement relatif entre les premier et second éléments allongés (666, 668) fait passer la première plaque d'extrémité (540) d'une première orientation angulaire à une seconde orientation angulaire, et dans lequel le dispositif de fixation spinale comprend en outre un ensemble de montage (600) soutenant de manière opérationnelle les premier et second éléments allongés (666, 668) dans le boîtier extérieur (510),
**caractérisé en ce que** l'ensemble de montage (600) inclut un boîtier de retenue (602) et des premier et second éléments rotatifs (610, 612) soutenus de manière rotative dans le boîtier de retenue (602), les premier et second éléments rotatifs (610, 612) étant respectivement accouplés de manière opérationnelle aux premier et second éléments allongés (666, 668) de l'ensemble de plaque d'extrémité (560).

2. Dispositif de fixation spinale selon la revendication 1, dans lequel l'ensemble de montage (600) est fixé de manière amovible au boîtier extérieur (510).

3. Dispositif de fixation spinale selon la revendication 1 ou 2, dans lequel les premier et second éléments rotatifs (610, 612) de l'ensemble de montage (600) sont espacés l'un de l'autre pour définir un espace entre eux.

4. Dispositif de fixation spinale selon la revendication 1, 2 ou 3, dans lequel chacun des premier et second éléments rotatifs (610, 612) de l'ensemble de montage (600) inclut des dents agencées circonférentiellement se faisant face.

5. Dispositif de fixation spinale selon l'une quelconque des revendications précédentes, dans lequel le premier élément rotatif (610) de l'ensemble de montage (600) inclut un filetage intérieur dans une première orientation et le second élément rotatif (612) de l'ensemble de montage (600) inclut un filetage intérieur dans une seconde orientation opposée à la première orientation.

6. Dispositif de fixation spinale selon la revendication 3, 4 ou 5, dans lequel le boîtier extérieur (510) définit une pluralité d'orifices, au moins un orifice de la pluralité d'orifices étant en communication avec l'espace défini entre les premier et second éléments rotatifs (610, 612) de l'ensemble de montage (600).

7. Dispositif de fixation spinale selon la revendication 6, dans lequel la pluralité d'orifices est agencée circonférentiellement autour du boîtier extérieur (510), ou
dans lequel la pluralité d'orifices est agencée le long d'une longueur du boîtier extérieur (510).

8. Kit de chirurgie spinale comprenant :
un dispositif de fixation spinale (500) selon l'une quelconque des revendications précédentes ; et
un instrument chirurgical définissant un canal s'étendant à travers, l'instrument chirurgical définissant une partie de mise en prise configurée pour fermement venir en prise avec le boîtier extérieur du dispositif de fixation spinale.

9. Kit de chirurgie spinale selon la revendication 8 lorsqu'elle est dépendante de la revendication 4, dans lequel l'instrument chirurgical inclut une commande de réglage de la hauteur (7000) s'étendant de manière rotative à travers le canal de l'instrument chirurgical, la commande de réglage de la hauteur (7000) incluant une partie de mise en prise ayant des dents configurées pour venir en prise avec les dents agencées circonférentiellement des premier et second éléments rotatifs (610, 612) de l'ensemble de montage (600), de sorte qu'une rotation de la commande de réglage de la hauteur (7000) entraîne une rotation des premier et second éléments rotatifs (610, 612) de l'ensemble de montage (600), et/ou
dans lequel l'instrument chirurgical inclut en outre une commande de réglage de l'angle (8000) s'étendant de manière rotative à travers le canal de l'instrument chirurgical, la commande de réglage de l'angle (8000) incluant une partie de mise en prise ayant des dents configurées pour venir en prise avec les dents agencées circonférentiellement d'un des premier ou second éléments rotatifs (610, 612) de l'ensemble de montage (600), de sorte qu'une rotation de la commande de réglage de l'angle (8000) entraîne une rotation de l'un des premier ou second éléments rotatifs (610, 612) de l'ensemble de montage (600).

10. Kit de chirurgie spinale selon la revendication 8 ou 9, comprenant un raccord à tige (1000) en plus ou à la place de l'instrument chirurgical, le raccord à tige (1000) ayant une partie d'ancrage accouplée au boîtier extérieur du dispositif de fixation spinale (500) et une partie de tête ayant une partie évidée configurée pour recevoir une tige spinale, de préférence
dans lequel le raccord à tige (1000) définit un canal à travers, plus préférentiellement
dans lequel la partie de tête du raccord à tige (1000) définit un orifice en communication avec le canal.

11. Kit de chirurgie spinale selon la revendication 8, 9 ou 10, comprenant en outre un ensemble d'administration de médicament en plus ou à la place de l'instrument chirurgical, l'ensemble d'administration de médicament incluant un réservoir de médicament fermement accouplé au boîtier extérieur (510) du dispositif de fixation spinale (500), un orifice d'amenée, et un cathéter en communication fluidique avec l'orifice d'amenée et le réservoir de médicament.

12. Kit de chirurgie spinale selon la revendication 8, 9, 10 ou 11, comprenant en outre un écran (4000) en plus ou à la place de l'instrument chirurgical, l'écran pouvant être attaché au dispositif de fixation spinale (500), de préférence
dans lequel l'écran (4000) est configuré pour réduire un rayonnement vers la moelle épinière, plus préférentiellement
dans lequel l'écran (4000) inclut un médicament à libération lente.
